# DEMANDE DE BREVET EUROPEEN

(11) **EP 4 822 333 A1**
(43) Date de publication de la demande: **14.10.2026**
(21) Numéro de dépôt: 25169278.6
(22) Date de dépôt: 08.04.2025
(51) Int. Cl.: G01N 15/08, A61L 2/28, B01D 46/00, B01D 65/10

(54) **DISPOSITIF POUR TEST D'INTEGRITE DE FILTRE STERILISANT**

(71) Demandeur: Trasis S.A., 4430 Ans (BE)
(72) Inventeur: MORELLE, Jean-Luc, 4000 LIEGE (BE); SIMILON, Olivier, 4430 ANS (BE); HORION, Arnaud, 4430 ANS (BE)
(74) Mandataire: AWA Benelux

(57) **Abrégé**

La présente invention divulgue un dispositif de test d'intégrité automatisé d'un filtre stérilisant hydrophile avec une pression théorique admissible comprenant :
- une seringue et un filtre stérilisant à tester, connectés l'un à l'autre via un chemin fluidique;
- un actionneur de seringue apte à appliquer en utilisation une pression sur ladite seringue ;
- un moyen permettant de mesurer en utilisation la consommation de courant dudit actionneur ;
- un contrôleur apte à générer la courbe de force de l'actionneur sur la seringue sur base des données collectées par ledit moyen permettant de mesurer ladite consommation et à calculer la pression de bulle effective du filtre sur base de la valeur la force maximale mesurée pendant le mouvement de la seringue, pour la comparer avec la pression de bulle théorique du filtre.

## Description

### Objet de l'invention

La présente invention se rapporte au domaine des dispositifs de test d'intégrité des filtres, en particulier de filtres stérilisants, dont l'intégrité est vérifiée grâce à la méthode dite du « point bulle ». Le dispositif a pour but de pouvoir réaliser le test de manière automatisée sur un synthétiseur.

### Etat de la technique

Dans le domaine de l'imagerie médicale, la préparation des radiotraceurs est une étape importante. Du fait de leur radioactivité, les radiotraceurs sont préparés et manipulés à l'aide de synthétiseurs et de systèmes de distribution automatisés au maximum. Lors de la production des solutions contenant ces radiotraceurs, des filtres stérilisants sont utilisés afin de garantir la stérilité de la solution produite, et donc de la sécurité du patient. Les agences de réglementation telles que la FDA (Food and Drug Administration) et l'EMA (Agence européenne des médicaments) exigent des tests d'intégrité des filtres systématiquement après chaque production et avant la libération des lots de radiotraceurs afin de s'assurer de leur intégrité. Les tests d'intégrité sont effectués pour s'assurer que le filtre stérilisant répond aux spécifications et que la membrane filtrante était intacte pendant la filtration.

Un filtre est considéré comme intègre si la membrane de celui-ci n'a pas été percée ou décollée. En cas de filtre non intègre, la production doit être rejetée. Pour cette raison, certains centres utilisent deux filtres pour limiter le risque de rejet d'une production.

A l'heure actuelle, il existe plusieurs méthodes non destructives pour tester l'intégrité d'un filtre hydrophile comme par exemple, le « test du point bulle », le « test du maintien en pression » et le « test de diffusion ». Le « test du point bulle » consiste à mettre le filtre hydrophile humide sous pression jusqu'à atteindre la pression permettant de vaincre la tension superficielle du liquide piégé dans la membrane du filtre et de libérer le passage de l'air à travers le filtre. La pression à laquelle l'air traverse le filtre est appelée « point bulle ». En fonction de la valeur du point bulle obtenu, le filtre sera considéré comme intègre ou non. Ensuite, le « test de maintien en pression » fait recours à l'utilisation d'un manomètre de précision afin de mesurer, en amont du filtre, les variations de pressions causées par la diffusion d'un gaz à travers le filtre. Ce test mesure la perte de pression du filtre humide à l'intérieur d'un boîtier sur une période donnée. Si la perte de pression est supérieure à une valeur spécifique, le filtre est considéré comme défectueux. Et enfin, le « test de diffusion » part du principe que lorsque la pression est inférieure à celle du point de bulle, les molécules de gaz se déplacent à travers les pores remplis d'eau d'une membrane mouillée suivant la loi de la diffusion (loi de Fick). Celle-ci stipule que, pour un filtre donné, le débit de diffusion est proportionnel à la pression appliquée et à la surface totale du filtre. Ce test consiste donc à mesurer le débit de gaz diffusant à travers la membrane du filtre humide à une pression d'environ 80 % de celle du point de bulle attendu. Si le débit du gaz de diffusion est supérieur à celui indiqué dans la documentation du filtre, l'élément filtrant est considéré comme défectueux.

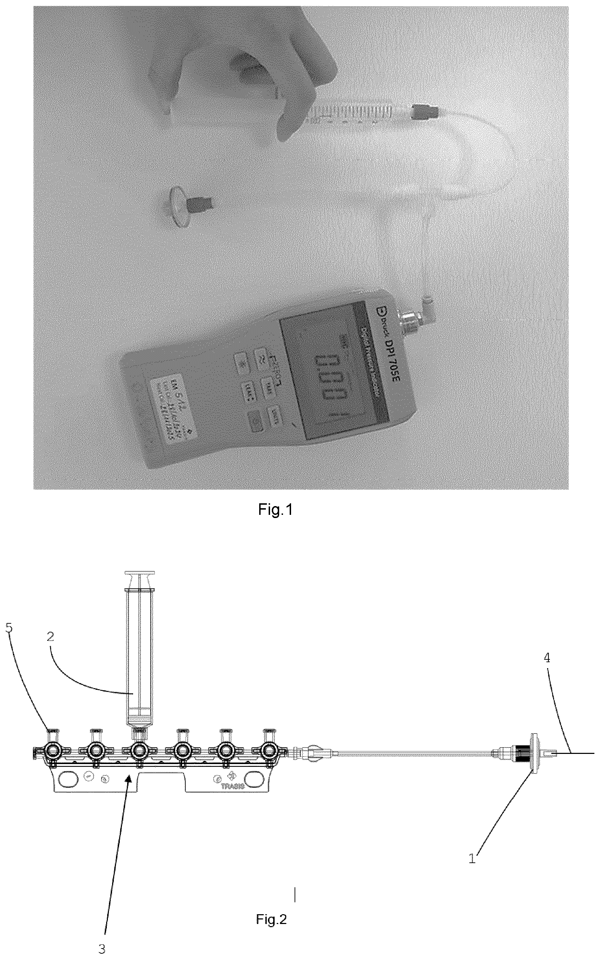

La majorité des systèmes actuels de vérification de l'intégrité des filtres stérilisants se basent sur le test du point bulle (voir par exemple le dispositif manuel de la Figure 1). Le test du point bulle n'est donc pas une mesure directe de la taille des pores, mais permet de déterminer l'intégrité du filtre à partir de la pression à laquelle la tension superficielle de l'eau à l'intérieur de la membrane filtrante est rompue. Lorsqu'elle est rompue en dessous de la valeur minimale spécifiée par le fabricant, cela signifie que le filtre est défectueux. En pratique, le filtre est d'abord conditionné, à savoir humidifié en faisant passer entre 5 et 10 ml de liquide adéquat à travers sa membrane (de l'eau saline en général, le résultat du test dépendant du liquide utilisé et renseigné par le fabricant du filtre). Ensuite, la pointe d'une aiguille placée à l'aval du filtre est placée dans un récipient contenant un liquide, usuellement de l'eau. Une pression via un gaz sur le filtre conditionné est alors appliquée, par exemple à l'aide d'une seringue contenant le gaz, et la pression à laquelle un train continu de bulles d'air apparait dans le liquide du récipient est observée. Cette pression (appelée « point bulle réel ») doit être supérieure à la valeur annoncée par le fabricant (appelée « point bulle théorique »).

Les automates de synthèses (synthétiseurs) permettant la synthèse automatisée de radiotraceurs comprennent en général une cassette avec un ensemble d'entrées et de sorties fluidiques ainsi que des seringues reliées à certaines desdites entrées afin d'injecter et de transférer le(s) produit(s) nécessaire(s). Pour injecter et transférer le produit de manière automatisée et précise, des pousse-seringues (actionneur) sont utilisés pour actionner les pistons des seringues. Un test de point bulle n'est pas réalisable sur les automates de synthèses car on ne sait pas détecter ce moment de début de fuite. La solution usuellement choisie consiste alors de procéder au test manuel du point de bulle tel que décrit dessus. Or, vu les temps de demi-vie relativement courts des isotopes intégrés aux radiotraceurs, ce test manuel doit se faire immédiatement après la production du radiotraceur. L'opérateur doit donc manipuler le filtre rapidement après la production, et se trouve dès lors fortement exposé aux radiations. Les tests de diffusion et de maintien en pression peuvent également être envisagés, mais sont également fortement exposants et sont moins fiables que le test de point bulle qui est la référence dans les directives des bonnes pratiques de fabrication.

Traditionnellement, les tests d'intégrité des filtres stériles sont donc effectués dans des dispositifs séparés du synthétiseurs. Autrement dit, le filtre stérile doit être retiré manuellement du synthétiseur pour effectuer le test d'intégrité. Cette approche traditionnelle pose un certain nombre de problèmes. Par exemple, il est conseillé d'attendre avant de tester l'intégrité du filtre afin que la radioactivité du résidu de radiotraceur dans le filtre soit moindre pour éviter toute contamination et irradiation de l'opérateur. Cependant, étant donné qu'un radiotraceur à courte durée de vie doit être injecté au patient rapidement après avoir été produit, un test d'intégrité différé serait trop tardif pour empêcher l'injection d'un lot défectueux. La seule alternative est alors que le personnel teste le filtre immédiatement après une synthèse, risquant ainsi de s'exposer à de fortes radiations.

Le document US 7, 770, 434 B2 divulgue une méthode permettant de réaliser des tests d'intégrité sur des filtres directement au cours du processus de la synthèse de radiotraceurs. Comme précédemment, une source d'air ou d'autre gaz est fournie, permettant d'injecter un gaz à travers le filtre et de contrôler la pression du gaz. Le système comprend également une source de lumière afin de faire passer un faisceau lumineux à travers une partie du tube, et un détecteur du faisceau lumineux qui permet de détecter et d'indiquer si le liquide dans le tube a subi un déplacement de part une ou plusieurs bulles de gaz. La pression pour laquelle le déplacement est observé dans le liquide correspond à la pression du point de bulle. La méthode de ce document est uniquement basée sur une détection optique et donc visuelle de l'apparition des bulles dans le liquide du tube.

### Buts de l'invention

La présente invention vise à surmonter les inconvénients de l'état de la technique, et en particulier à fournir un dispositif permettant de tester l'intégrité d'un filtre hydrophile au cours du processus de synthèse du radiotraceur. Plus précisément, le dispositif de la présente invention a pour but d'être intégré au sein du synthétiseur des solutions radioactives, afin d'éviter de devoir manipuler le filtre du système de synthèse. Le filtre peut être testé directement après la production du radiotraceur, pour pouvoir injecter la solution rapidement étant donné la durée de vie de la charge radioactive. Comme le filtre ne doit pas être manipulé par un opérateur, il n'est pas nécessaire de différer le test pour limiter la dose en contact avec l'opérateur.

Un autre but de l'invention est de fournir une méthode simple, qui offre une précision, une répétabilité ainsi qu'une fiabilité accrues par rapport aux dispositifs de l'art antérieur.

Un autre but de l'invention est d'offrir une solution compacte, qui peut s'intégrer aux synthétiseurs, qu'ils soient nouveaux ou existants, pour tester l'intégrité du filtre sans le retirer du système.

### Résumé de l'invention

La présente invention divulgue un dispositif de test d'intégrité automatisé d'un filtre stérilisant hydrophile avec une pression théorique admissible comprenant :
- une seringue et un filtre stérilisant à tester, connectés l'un à l'autre via un chemin fluidique;
- un actionneur de seringue apte à appliquer en utilisation une pression sur ladite seringue ;
- un moyen permettant de mesurer en utilisation la consommation de courant dudit actionneur ;
- un contrôleur apte à générer la courbe de force de l'actionneur sur la seringue sur base des données collectées par ledit moyen permettant de mesurer ladite consommation et à calculer la pression de bulle effective du filtre sur base de la valeur la force maximale mesurée pendant le mouvement de la seringue, pour la comparer avec la pression de bulle théorique du filtre.

Selon des modes d'exécution particuliers, l'invention comporte une ou plusieurs des caractéristiques suivantes :
- le dispositif est intégré à un synthétiseur automatisé de solutions radioactives ce qui permet de ne pas retirer le filtre du synthétiseur pour effectuer le test ;
- le chemin fluidique entre la seringue et le filtre à tester est la cassette à usage unique utilisée pour produire la solution radioactive.

La présente invention concerne également une méthode de test automatisé de l'intégrité d'un filtre stérilisant hydrophile, le filtre à tester étant connecté à une seringue remplie d'un gaz, la méthode comprenant les étapes suivantes :
- conditionnement d'un filtre en faisant passe une solution aqueuse à travers la membrane du filtre ;
- mise sous pression de la membrane du filtre à l'aide d'un actionneur de seringue actionnant une seringue remplie d'un gaz ;
- prise de mesure de la consommation de courant de l'actionneur ;
- établissement de la courbe de force appliquée par l'actionneur sur la seringue ;
- calcul du point bulle effectif sur base de la force maximale mesurée pendant le mouvement de la seringue.

Selon des modes d'exécution particuliers, la méthode de l'invention comporte une ou plusieurs des caractéristiques suivantes :
- l'actionneur de seringue actionne la seringue à une vitesse constante permettant un débit compris entre 5 et 25 mL/min ;
- le conditionnement du filtre est réalisé par le passage de 3 à 10 ml de solution aqueuse ;
- la solution aqueuse pour conditionner le filtre est de l'eau saline ;
- le gaz contenu dans la seringue est de l'air ;
- une étape de comparaison de la valeur du point de bulle effectif avec la valeur théorique du filtre est réalisée pour décider de la conformité du filtre, si la pression effective est inférieure à la pression théorique, le filtre est considéré comme non conforme et si la pression effective est supérieure ou égale à la pression théorique, le filtre est considéré comme conforme ;
- la méthode est couplée à une étape de synthèse d'une solution radioactive sur un synthétiseur, le filtre stérilisant hydrophile utilisé lors de la synthèse précédent la méthode de test étant testé lors de la méthode de test ;
- le filtre n'est pas enlevé du synthétiseur pour les étapes de test d'intégrité.

### Brève description des figures

La figure 1 représente l'appareillage pour un test du point de bulles selon l'art antérieur, effectué manuellement.
La figure 2 représente un exemple non limitatif d'un assemblage des différents éléments amovibles requis pour le test de point de bulles automatisé sur un synthétiseur à cassette suivant la présente invention.
La figure 3 est un diagramme représentant la courbe de force du pousse-seringue lors de la montée en pression sur le filtre à un débit de 25 ml/min.
La figure 4 est un diagramme représentant la courbe de force du pousse-seringue lors de la montée en pression sur le filtre à un débit de 10 ml/min.

### Liste des symboles de référence

- 1 :: filtre hydrophile à tester
- 2 :: seringue
- 3 :: cassette
- 4 :: aiguille
- 5 :: entrée de la cassette
- 6 :: pousse-seringue (actionneur)

### Description détaillée de l'invention

Le test d'intégrité du filtre de la présente invention repose sur le principe du point bulle (ou « PB » par la suite, ou « BP » en anglais). Comme expliqué précédemment, le PB d'un filtre hydrophile est la pression appliquée sur la membrane humide du filtre au-dessus de laquelle un flux continu de bulles commence à passer à travers la membrane. La valeur de pression mesurée est le PB effectif (ou PB mesuré). La valeur donnée par le fabricant du filtre est le PB théorique. Les filtres dont la pression effective est inférieure à la pression théorique sont considérés comme des filtres non conformes.

La valeur PB effectif dépend du fluide utilisé pour mouiller le filtre. La valeur théorique d'un filtre est toujours donnée pour un fluide donné, spécifié dans la fiche technique (généralement de l'eau pour une membrane hydrophile). La valeur théorique du filtre pour un autre fluide ( ${BP}_{fluid}^{th}$) peut être déterminée par la formule suivante : ${BP}_{fluid}^{th} = {BP}_{water}^{th} ∗ \left(\frac{{BP}_{fluid}^{measured}}{{BP}_{water}^{measured}}\right)$

Le dispositif de la présente invention permet avantageusement l'obtention de la valeur du PB effectif de manière automatisée, sans que le filtre ne doive être enlevé de l'automate de synthèse des radiotraceurs (contrairement au test du point de bulles de l'art antérieur tel que représenté sur la figure 1). L'intégrité du filtre est donc testée au cours du processus de production. La valeur du PB effectif est obtenue de manière innovante sur base de la mesure instantanée et continue de la force appliquée sur le pousse-seringue permettant d'injecter le gaz sur le filtre et donc de mettre ce dernier sous pression via l'actionnement de la seringue.

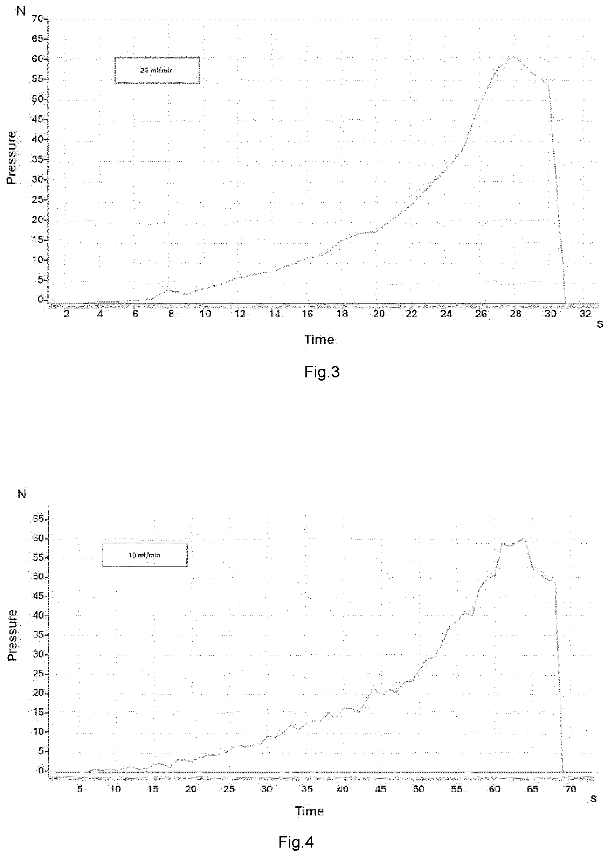

Le dispositif de test d'intégrité automatisé de la présente invention est illustré à la figure 2. Celui-ci comprend une seringue 2 et un filtre stérilisant à tester 1, connectés l'un à l'autre via un chemin fluidique. Un actionneur de seringue 6 est prévu afin d'appliquer en utilisation une pression sur la seringue. Pour obtenir la valeur du PB effectif, un moyen mesure la consommation de courant consommé par l'actionneur du pousse-seringue. Le moyen de mesure de la consommation peut être par exemple la carte de contrôle du moteur qui est capable de donner l'information en temps réel du courant consommé par l'actionneur. La valeur de la mesure de consommation de courant instantané permet de calculer la force appliquée sur le pousse-seringue. De cette manière, une courbe de force du pousse-seringue est obtenue, tel qu'illustrée sur les diagrammes des figures 3 et 4. Ces diagrammes représentent la force appliquée sur le pousse-seringue par rapport au temps, force qui est facilement convertie en pression appliquée par le gaz sur la membrane du filtre. Comme illustré sur les diagrammes, la force augmente petit à petit jusqu'à une chute brutale. Il a été observé par les inventeurs que le PB effectif se produit à la force maximale appliquée, juste avant la chute de force. La chute de force correspond à la chute de pression sur la membrane du filtre au moment où le flux d'air passe à travers la membrane, et donc au moment où la première bulle et ensuite le train de bulles se formeraient dans le liquide dans le cas où le filtre serait relié à une aiguille placée dans un récipient rempli de liquide.

Sur la figure 3, le gaz qui est injecté sur le filtre a un débit de 25 ml/min, tandis que sur la figure 4, le débit du gaz qui est injecté par la seringue sur le filtre est de 10ml/min. Dans les deux cas, la pression est augmentée de manière substantiellement constante jusqu'à l'observation d'une première chute légère correspondant à l'apparition des premières bulles, avant une chute brutale correspondant au passage continu d'air par la formation du train de bulles dans le cas où le filtre serait relié à une aiguille placée dans un récipient rempli de liquide. Les courbes montrent respectivement un PB réel de 61 N et 60 N, supérieur dans les deux cas au point bulle théorique des filtres testés (25 N). On conclut que les filtres étaient bien intègres.

De par cette analyse, le dispositif de la présente invention a pu être mis en oeuvre, et offrir la possibilité de fournir la valeur du PB effectif grâce à la mesure du courant consommé par l'actionneur du pousse-seringue. De cette manière, le dispositif de la présente invention permet l'obtention de la valeur du PB effectif de manière automatisée et sans que le filtre ne doive être enlevé de l'automate de synthèse, ou ne doive être relié à une aiguille trempée dans un récipient d'eau pour observer la formation des bulles. Ceci a pour principal avantage d'éviter une intervention manuelle de la part d'un opérateur, ce qui l'exposerait à la radioactivité.

Les séquences détaillées, effectuées par un automate de synthèse en fin de production, du test d'intégrité du filtre automatisé de la présente invention sont les suivantes :
- le filtre est conditionné en faisant passer une solution aqueuse à travers la membrane, de préférence de 3 à 10 ml de solution saline ;
- un actionneur de seringue pousse une seringue à une vitesse, par exemple entre 5 et 25 mL/min, appliquant une augmentation de pression, de préférence à vitesse constante, sur la membrane filtrante ;
- la consommation de courant de l'actionneur est mesurée, ce qui permet d'en déduire la courbe de force et donc de pression appliquée par l'actionneur sur la seringue (par multiplication de la mesure par un facteur d'étalonnage pression/courant) ;
- la pression maximale mesurée pendant le mouvement de la seringue est enregistrée et correspond au PB effectif ;
- la valeur du PB effectif est comparée avec la valeur du PB théorique du filtre, généralement annoncé par le fabricant. Si la pression effective est inférieure à la pression théorique, le filtre est considéré comme non conforme. Si la pression effective est supérieure ou égale à la pression théorique, le filtre est considéré comme conforme.

Si le filtre est considéré comme non conforme lors de la séquence de test d'intégrité, l'utilisateur devra à nouveau humidifier le filtre et effectuer un test d'intégrité manuel.

Les séquences du test d'intégrité de la présente invention sont réalisées sans devoir enlever le filtre du système de synthèse. Tous les éléments utilisés pour le bon déroulement du test sont prévus au sein du synthétiseur, ou sont couplés au synthétiseur de sorte que le test se fasse lors de la production des traceurs radiopharmaceutiques.

La méthode est conforme aux pharmacopées européenne et américaine. La méthode a été validée en utilisant deux appareils différents utilisés par des utilisateurs différents, avec un lot de 50 filtres testés par type d'équipement. Une proportion de 10 % de filtres défectueux a été introduite intentionnellement parmi les 50 filtres. Le test a permis d'identifier 100 % des filtres non endommagés comme étant conformes, tout en évitant les résultats faussement positifs et faussement négatifs en tenant compte de la précision de l'actionneur de la seringue.

Le dispositif de test de la présente invention comprend donc :
- une seringue et un filtre stérilisant connectés l'un à l'autre via un chemin fluidique ;
- un actionneur de seringue apte à appliquer en utilisation une augmentation de pression sur la membrane du filtre à tester ;
- un moyen permettant de mesurer en utilisation la consommation de courant de l'actionneur ;
- un contrôleur pour générer la courbe de force appliqué par l'actionneur sur la seringue sur base des données de consommation de courant collectées par le moyen permettant de mesurer ladite consommation et calculer le PB effectif sur base de la valeur de la force maximale mesurée pendant le mouvement de la seringue, afin de le comparer avec le PB théorique fabricant. Un collecteur peut éventuellement être prévu, avec au moins deux entrées, permettant la connexion du filtre à tester en aval du collecteur.

La présente invention n'est pas limitative au chemin fluidique entre la seringue et le filtre stérilisant. Le chemin fluidique entre la seringue et le filtre stérilisant peut par exemple et avantageusement être celui d'une cassette à usage unique placée sur un automate de synthèse à cassette. Dans ce cas, la seringue st actionnée automatiquement par l'automate. Dans une autre application de l'invention, il peut s'agir d'une tubulure souple connectée d'une part à une seringue montée sur un pousse seringue indépendant et, d'autre part, au filtre stérilisant.

## Revendications

1. Dispositif de test d'intégrité automatisé d'un filtre stérilisant hydrophile avec une pression théorique admissible comprenant :
- une seringue (2) et un filtre stérilisant (1) à tester, connectés l'un à l'autre via un chemin fluidique;
- un actionneur de seringue (6) apte à appliquer en utilisation une pression sur ladite seringue (2) ;
- un moyen permettant de mesurer en utilisation la consommation de courant dudit actionneur (6);
- un contrôleur apte à générer la courbe de force de l'actionneur (6) sur la seringue (2) sur base des données collectées par ledit moyen permettant de mesurer ladite consommation et à calculer la pression de bulle effective du filtre ( 1) sur base de la valeur la force maximale mesurée pendant le mouvement de la seringue (2), pour la comparer avec la pression de bulle théorique du filtre (1).

2. Dispositif de test selon la revendication 1, **caractérisé en ce qu'**il est intégré à un synthétiseur automatisé de solutions radioactives, permettant de ne pas retirer le filtre (1) du synthétiseur pour effectuer le test.

3. Dispositif de test selon la revendication 2, **caractérisé en ce que** le chemin fluidique entre la seringue (2) et le filtre à tester (1) est une cassette à usage unique (3) utilisée pour produire la solution radioactive.

4. Méthode de test automatisé de l'intégrité d'un filtre stérilisant hydrophile, le filtre à tester (1) étant connecté à une seringue (2) remplie d'un gaz, ladite méthode comprenant les étapes suivantes :
- conditionnement d'un filtre (1) en faisant passe une solution aqueuse à travers la membrane du filtre (1) ;
- mise sous pression de la membrane du filtre (1) à l'aide d'un actionneur de seringue (6) actionnant une seringue (2) remplie d'un gaz ;
- prise de mesure de la consommation de courant de l'actionneur (6) ;
- établissement de la courbe de force appliquée par l'actionneur (6) sur la seringue (2) ;
- calcul du point bulle effectif sur base de la force maximale mesurée pendant le mouvement de la seringue (2).

5. Méthode de test automatisé selon la revendication 4, **caractérisé en ce que** l'actionneur de seringue (6) actionne la seringue (2) à une vitesse constante permettant un débit compris entre 5 et 25 mL/min.

6. Méthode de test automatisé selon l'une des revendications 4 à 5, **caractérisé en ce que** le conditionnement du filtre (1) est réalisé par le passage de 3 à 10 ml de solution aqueuse.

7. Méthode de test automatisé selon la revendication 6, **caractérisé en ce que** la solution aqueuse pour conditionner le filtre (1) est de la saline.

8. Méthode de test automatisé selon l'une des revendications 4 à 7, **caractérisé en ce que** le gaz contenu dans la seringue (2) est de l'air.

9. Méthode de test automatisé selon l'une des revendications 4 à 8, **caractérisé en ce qu'**une étape de comparaison de la valeur du point de bulle effectif avec la valeur théorique du filtre (1) est réalisée pour décider de la conformité du filtre (1), si la pression effective est inférieure à la pression théorique, le filtre (1) est considéré comme non conforme et si la pression effective est supérieure ou égale à la pression théorique, le filtre (1) est considéré comme conforme.

10. Méthode de test automatisé selon les revendications 4 à 9 **caractérisé en ce qu'**elle est couplée à une étape de synthèse d'une solution radioactive sur un synthétiseur, le filtre stérilisant hydrophile (1) utilisé lors de la synthèse précédent la méthode de test étant testé lors de la méthode de test.

11. Méthode de test automatisé selon la revendication 10 **caractérisée en ce que** le filtre (1) n'est pas enlevé du synthétiseur pour les étapes de test d'intégrité.
